# EUROPEAN PATENT SPECIFICATION

(11) **EP 3 004 219 B1**
(45) Date of publication and mention of the grant of the patent: **04.04.2018**
(21) Application number: 14728784.1
(22) Date of filing: 12.05.2014
(51) Int. Cl.: C08J 3/03, C08G 77/26, C08L 83/08, A61K 8/41, A61Q 19/00, A61K 8/06, A61K 8/891, A61K 8/898

(54) **PROCESS FOR PREPARING AMINOFUNCTIONAL SILICONE EMULSIONS**
VERFAHREN ZUR HERSTELLUNG VON AMINOFUNKTIONELLEN SILIKONEMULSIONEN
PROCÉDÉ DESTINÉ À LA PRÉPARATION D'ÉMULSIONS DE SILICONE AMINOFONCTIONELLE

(30) Priority: 31.05.2013 US 201361829366 P
(43) Date of publication of application: 13.04.2016
(73) Proprietor: Dow Corning Corporation, Midland, Michigan 48686-0994 (US)
(72) Inventor: ZIMMERMAN, Brett, Lee, Frankenmuth, MI 48734 (US)
(74) Representative: Murgitroyd & Company
(86) International application number: PCT/US2014/037628
(87) International publication number: WO 2014/193636

(56) References cited:
- WO-A1-00/49090
- WO-A1-2012/012524
- "Luviquat® Mono LS: Cationic quaternary ammonium salt for use as a conditioning agent, emulsifier and solubilizer", BASF Technical Information, September 2005 (2005-09), pages 1-6, XP002727647, Retrieved from the Internet: URL:http://dewolfchem.com/wp-content/uploa ds/2013/08/Luviquat-Mono-LS.Tech-Sheet.09. 01.05.pdf [retrieved on 2014-06-23]

## Description

### BACKGROUND

Emulsions of aminofunctional silicones and high molecular weight silicones are widely used in hair care compositions to provide various aesthetic benefits. Various types of emulsions have been commercially developed to provide water based products of such aminofunctional silicone polymers for use as hair conditioning agents.

Reducing the presence of solvents, un-reacted siloxanes, catalyst residues, cyclic polymerization byproducts, and other impurities in silicone emulsions is an ongoing challenge in the art. The need to reduce such impurities may arise, among other reasons, when such impurities are incompatible with downstream applications (for example, medical, cosmetic, and personal care applications), where the presence of such impurities would reduce the stability of an emulsion, or where regulatory requirements require removal or reduction of their presence. In particular, there is an interest to reduce the presence of cyclosiloxanes, such as octamethylcyclotetrasiloxanes and decamethylcyclopentasiloxanes, in silicone emulsions.

WO2012/012524 discloses a process for producing mechanical emulsions of aminofunctional siloxanes having reduced content of cyclosiloxanes. More specifically, WO2012/012524 discloses a process for preparing mechanical emulsions of aminofunctional siloxanes using a halide free quaternary ammonium surfactant. The amount of octamethylcyclotetrasiloxanes and decamethylcyclopentasiloxanes in the emulsions produced by WO2012/012524 process were reduced when compared to emulsions prepared by conventional methods.

The present inventor has discovered further improvements of the process disclosed in WO2012/012524. These improvements provide an enhancement of the storage stability of the emulsions of aminofunctional siloxanes.

### BRIEF SUMMARY

WO2012012524 discloses the use of a halide free quaternary ammonium surfactant reduces the extent of re-equilibration (cyclic siloxane generation) occurring in this type of emulsion. The present inventor has discovered an improvement to the WO2012012524 process for preparing aminofunctional silicone emulsions. It has been found that the type of neutralization agent is critical for complete chemical stability of the aminofunctional silicone emulsion. In particular, the present inventor has discovered that using a neutralizing agent with the same counter-ion as that contained on the quaternary ammonium surfactant improves the chemical stability of the aminofunctional silicone emulsion by further minimizing the generation of cyclic siloxanes upon storage.

The present disclosure provides a process for preparing an aminofunctional silicone emulsion comprising:
I) forming a mixture of;
   A) 100 parts of a polydialkylsiloxane having a viscosity of
      at least 50,000 mm²/s at 23°C as determined by a Brookfield Cone and Plate Viscometer Model DVT, cone #CP 52, at 0.5 rpm, and
   B) 0.1 to 100 parts of an aminofunctional organopolysiloxane having an average formula;

      [R₃SiO_{1/2}][R₂SiO_{2/2}]ₐ[RR^{N}SiO_{2/2}]_{b}[R₃SiO_{1/2}]

      where; a is 1-1000, b is 1-100,
      R is independently a monovalent organic group, and
      R^{N} is an amino functional group.
II) admixing to the mixture of step I;
   C) 0.1 to 50 parts of a halide free quaternary ammonium surfactant of the
      formula; R⁵ₐR⁶₍₄₋ₐ)N⁺ X⁻, wherein
      a varies from 1 to 4,
      R⁵ is an organic group containing at least 10 carbon atoms,
      R⁶ is independently a hydrocarbon group containing 1 to 20 carbon atoms, X is a halide free counter ion selected from methosulfate, and
      a sufficient amount of water to form an emulsion,
III) optionally, further shear mixing the emulsion,
IV) adding a sufficient amount of an acid of the formula H⁺ X⁻ to provide the emulsion with a neutral pH in the range of 6.5 to 7.5 at 23°C, where X is the same counter ion used in the quaternary ammonium surfactant.

### DETAILED DESCRIPTION

The first step in the present process involves forming a mixture of;
A) 100 parts of a polydialkylsiloxane having a viscosity of
   at least 50,000 mm²/s at 23°C as determined by a Brookfield Cone and Plate Viscometer Model DVT, cone #CP 52, at 0.5 rpm, and
B) 0.1 to 100 parts of an aminofunctional organopolysiloxane
   having an average formula;

      [R₃SiO_{1/2}][R₂SiO_{2/2}]ₐ[RR^{N}SiO_{2/2}]_{b}[R₃SiO_{1/2}]

      where; a is 1-1000, b is 1-100,
      R is independently a monovalent organic group, and
      R^{N} is an amino functional group.

### A) The Polydialkylsiloxane

Component A) in the present process is a polydialkylsiloxane. Component A) may be selected from polydialkylsiloxanes having the general formula;

[R¹₂R²SiO_{1/2}][R¹₂SiO_{2/2}]_{x[}R¹₂ R²SiO_{1/2}]

where R¹ is an alkyl group containing 1 to 30 carbon atoms, R² may be an R¹ alkyl group or a hydroxy group, the subscript "x" represents the degree of polymerization and is greater than 1000. Typically, the polydialkylsiloxane is a trimethylsiloxy terminated polydimethylsiloxane fluid having a degree of polymerization (x) that is sufficient to provide a polydimethylsiloxane fluid viscosity of at least 50,000 mm²/s (or 50,000 centistokes, abbreviated as cS) at 23°C, alternatively (x) is sufficient to provide a polydimethylsiloxane fluid viscosity of at least 100,000 mm²/s at 23°C alternatively (x) is sufficient to provide a polydimethylsiloxane fluid viscosity of at least 500,000 mm²/s at 23°C. Representative commercial products of trimethylsiloxy terminated polydimethylsiloxane fluids suitable as component A) include Dow Corning 200® fluids (Dow Corning Corporation, Midland MI) having a viscosity of at least 50,000 centistokes.

The polydialkylsiloxane may also be a mixture of various polydialkylsiloxanes. Furthermore, the polydialkylsiloxane may also be dissolved in a suitable solvent, such as a lower molecular weight (that is where x is less than 1000) polydialkylsiloxane.

### B) The Aminofunctional Organopolysiloxane

Organopolysiloxanes are polymers containing siloxane units independently selected from (R₃SiO_{1/2}), (R₂SiO_{2/2}), (RSiO_{3/2}), or (SiO_{4/2}) siloxy units, where R may be any monovalent organic group. When R is a methyl group in the (R₃SiO_{1/2}), (R₂SiO_{2/2}), (RSiO_{3/2}), or (SiO_{4/2}) siloxy units of an organopolysiloxane, the siloxy units are commonly referred to as M, D, T, and Q units respectively. These siloxy units can be combined in various manners to form cyclic, linear, or branched structures. The chemical and physical properties of the resulting polymeric structures can vary. For example organopolysiloxanes can be volatile or low viscosity fluids, high viscosity fluids/gums, elastomers or rubbers, and resins depending on the number and type of siloxy units in the average polymeric formula. R may be any monovalent organic group, alternatively R is a hydrocarbon group containing 1 to 30 carbons, alternatively R is an alkyl group containing 1 to 30 carbon atoms, or alternatively R is methyl.

The aminofunctional organopolysiloxanes of the present disclosure are characterized by having at least one of the R groups in the formula RₙSiO_{(4-n)/2} be an amino group. The amino functional group may be present on any siloxy unit having an R substituent, that is, they may be present on any (R₃SiO_{1/2}), (R₂SiO_{2/2}), or (RSiO_{3/2}) unit, and is designated in the formulas herein as R^{N}. The amino-functional organic group R^{N} is illustrated by groups having the formula; -R³NHR⁴, -R³NR₂⁴, or-R³NHR³NHR⁴, wherein each R³ is independently a divalent hydrocarbon group having at least 2 carbon atoms, and R⁴ is hydrogen or an alkyl group. Each R³ is typically an alkylene group having from 2 to 20 carbon atoms. R³ is illustrated by groups such as; -CH₂CH₂-, -CH₂CH₂CH₂-, -CH₂CHCH₃-, -CH₂CH₂CH₂CH₂-, - CH₂CH(CH₃)CH₂-, -CH₂CH₂CH₂CH₂CH₂-, -CH₂CH₂CH₂CH₂CH₂CH₂-, - CH₂CH₂CH(CH₂CH₃)CH₂CH₂CH₂-, -CH₂CH₂CH₂CH₂CH₂CH₂CH₂CH₂-, and -CH₂CH₂CH₂CH₂CH₂CH₂CH₂CH₂CH₂CH₂-. The alkyl groups R⁴ are as illustrated above for R. When R⁴ is an alkyl group, it is typically methyl.

Some examples of suitable amino-functional hydrocarbon groups are; -CH₂CH₂NH₂, -CH₂CH₂CH₂NH₂, -CH₂CH(CH₃)NH₂, -CH₂CH₂CH₂CH₂NH₂, -CH₂CH₂CH₂CH₂CH₂NH₂, -CH₂CH₂CH₂CH₂CH₂CH₂NH₂, -CH₂CH₂NHCH₃, -CH₂CH₂CH₂NHCH₃, -CH₂CH(CH₃)CH₂NHCH₃, -CH₂CH₂CH₂CH₂NHCH₃, -CH₂CH₂NHCH₂CH₂NH₂, - CH₂CH₂CH₂NHCH₂CH₂NH₂, -CH₂CH₂CH₂NHCH₂CH₂CH₂NH₂, -CH₂CH₂CH₂CH₂NHCH₂CH₂CH₂CH₂NH₂, -CH₂CH₂NHCH₂CH₂NHCH₃, -CH₂CH₂CH₂NHCH₂CH₂CH₂NHCH₃, -CH₂CH₂CH₂CH₂NHCH₂CH₂CH₂CH₂NHCH₃, and -CH₂CH₂NHCH₂CH₂NHCH₂CH₂CH₂CH₃.

Alternatively, the amino functional group is -

CH₂CH(CH₃)CH₂NHCH₂CH₂NH₂.

The aminofunctional organopolysiloxane used as component B) may be selected from those having the average formula;

[R₃SiO_{1/2}][R₂SiO_{2/2}]ₐ[RR^{N}SiO_{2/2}]_{b}[R₃SiO_{1/2}]

where; a is 1-1000, but also disclosed is where a is 1 to 500 or 1 to 200,
b is 1-100, but also disclosed is where b is 1 to 50 or 1 to 10,
R is independently a monovalent organic group,
   alternatively R is a hydrocarbon containing 1- 30 carbon atoms,
   alternatively R is a monovalent alkyl group containing 1 - 12 carbons, or
   alternatively R is a methyl group;
R^{N} is as defined above.

The aminofunctional organopolysiloxane used as component B) may also be a combination of any of the aforementioned aminofunctional organopolysiloxanes. The aminofunctional organopolysiloxane may also be dissolved in a suitable solvent, such as a lower molecular weight organopolysiloxane or organic solvent.

Mixing in step (I) can be accomplished by any method known in the art to effect mixing of high viscosity materials. The mixing may occur either as a batch, semi-continuous, or continuous process. Mixing may occur, for example using, batch mixing equipment with medium / low shear include change-can mixers, double-planetary mixers, conical-screw mixers, ribbon blenders, double-arm or sigma-blade mixers; batch equipments with high-shear and high-speed dispersers include those made by Charles Ross & Sons (NY), Hockmeyer Equipment Corp. (NJ); batch mixing equipment such as those sold under the tradename Speedmixer®; batch equipment with high shear actions include Banbury-type (CW Brabender Instruments Inc., NJ) and Henschel type (Henschel mixers America, TX). Illustrative examples of continuous mixers / compounders include extruders single-screw, twin-screw, and multi-screw extruders, co-rotating extruders, such as those manufactured by Krupp Werner & Pfleiderer Corp (Ramsey, NJ), and Leistritz (NJ); twin-screw counter-rotating extruders, two-stage extruders, twin-rotor continuous mixers, dynamic or static mixers or combinations of these equipments.

Step II) in the present process involves admixing;
C) 0.1 to 50 parts of a halide free quaternary ammonium surfactant containing at least 10 carbon atoms, and
   a sufficient amount of water to form an emulsion.

### C) The Quaternary Ammonium Surfactant

Component C) in the present process is a halide free quaternary ammonium surfactant containing at least 10 carbon atoms. As used herein "halide free" means the quaternary ammonium surfactant does not contain fluoride, chloride, bromide, or iodide as a counter-ion in the quaternary ammonium compound.

The halide free quaternary ammonium surfactant may have the formula;

R⁵ₐR⁶₍₄₋ₐ)N⁺ X⁻,

wherein
the subscript "a" may vary from 1 to 4, alternatively "a" is 1.
R⁵ is an organic group containing at least 10 carbon atoms,
R⁶ is independently a hydrocarbon group containing 1 to 20 carbon atoms,
X is a halide free counter ion.

In the above formula R⁵ is an organic group containing at least 10 carbon atoms. Representative, examples of R⁵ include alkyl groups such as decyl, undecyl, dodecyl, hexadecyl, octadecyl. R⁵ may also be selected from those organic groups considered as being derived from "fatty acids or fatty alcohols" such as lauryl, cetyl, coco, stearyl, tallow, cocoyl, lauroyl, palmitoyl, myristoyl or stearoyl. Alternatively, R¹ is a coco group.

R⁶ is independently a hydrocarbon group containing 1 to 20 carbon atoms. R⁶ may be an alkyl group, alkenyl group, aryl, or alkylaryl group. Alternatively R⁶ is an alkyl group containing 1 to 4 carbon atoms such as methyl, ethyl, propyl, or butyl. Alternatively R² is methyl or ethyl.

X is a halide free counter ion. In accordance with the invention , X is selected from methosulfate as possible counter ions. However, etho-sulfate, acetate, tosylate, phosphate, or nitrate are also disclosed as possible counter ions.

Representative examples of commercially available quaternary ammonium salts suitable in the present process include;

| Generic name | Commercial name |
|---|---|
| coco trimethylammonium methosulfate | Luviquat Mono LS |
| coco trimethylammonium methosulfate | Surtec 932 |
| coco trimethylammonium methosulfate | Servamine KAC458 |
| cetyl trimethylammonium methosulfate | Crodazosoft DBQ |
| stearyl trimethylammonium methosulfate | Empigen CM |
| cocoylcholine methosulfate | Surfactive VCC |
| lauroyl ethyltrimethylammonium methosulfate | Surfactive V 12 |
| myristoyl ethyltrimethylammonium methosulfate | Surfactive V 14 |
| palmitoyl ethyltrimethylammonium methosulfate | Surfactive V 16 |
| cetyl ethyldimethylammonium ethosulfate Etilsulfate | Mecetronium |
| coco ethyldimethylammonium ethosufalte | Dextrol AS-150 |
| cetyl trimethylammonium tosylate | Cetats |
| dimethyl PABAmidopropyl lauryldimethylammonium tosylate | Esccalol HP |
| bis-hydroxyethyl trimethylammonium nitrate Nitrate | Ethoquad C/12 |
| tallow trihydroxy ethylammonium acetate | Ethoquad T/13-50 |
| tallow trihydroxy ethylammonium acetate | Ethoquad T/13-27W |

An optional nonionic surfactant, designated herein as component D), may be also included in step II) of the present process. Some suitable nonionic surfactants which can be used include polyoxyethylene alkyl ethers, polyoxyethylene alkyl phenyl ethers, polyoxyethylene fatty acid esters, sorbitan fatty acid esters, and polyoxyethylene sorbitan fatty acid esters. Nonionic surfactants which are commercially available include compositions such as (i) 2,6,8-trimethyl-4-nonyl polyoxyethylene ether sold under the names Tergitol TMN-6 and Tergitol TMN-10; (ii) the C11-15 secondary alkyl polyoxyethylene ethers sold under the names Tergitol 15-S-7, Tergitol 15-S-9, Tergitol 15-S-15, Tergitol 15-S-30, and Tergitol 15-S-40, by the Dow Chemical Company, Midland, Michigan; octylphenyl polyoxyethylene (40) ether sold under the name Triton X405 by the Dow Chemical Company, Midland, Michigan; (iii) nonylphenyl polyoxyethylene (10) ether sold under the name Makon by the Stepan Company, Northfield, Illinois; (iv) ethoxylated alcohols sold under the name Trycol 5953 by Henkel Corp./Emery Group, Cincinnati, Ohio; and (v) ethoxylated alcohols sold under the name Brij by Uniqema (ICI Surfactants), Wilmington, Delaware, and ethoxylates of alkyl polyethylene glycol ethers based on the C10-Guerbet alcohol sold under the tradename Lutensol (BASF), such as Lutensol XP 79.

When the optional nonionic surfactant is used in step II), the amount may vary from 0.01 to 50 parts of the nonionic surfactant for every 100 parts of the polydialkylsiloxane used in the process.

Step II also involves the addition and mixing of water with the resulting mixture from step I), with component C) and optionally D). Typically 5 to 700 parts water are mixed for every 100 parts of the step I mixture to form an emulsion.

The mixing of the components in step II may be effected by the same mixing techniques as described above for step I). Mixing may also be effected using shear mixing techniques such as a rotor stator mixer, a homogenizer, a sonolator, a microfluidizer, a colloid mill, mixing vessels equipped with high speed spinning or with blades imparting high shear, or sonication to effect the formation of the emulsion.

In one example the emulsion formed is a water continuous emulsion. Typically, the water continuous emulsion has dispersed particles of the step I) mixture, and having an average particle size less than 1000 µm. Alternatively, the average volume particle size of the emulsions prepared according to the inventive process is between 0.05 µm and 1000 µm; or between 0.1 µm and 500 µm; or between 0.1 µm and 100 µm; or between 1 and 10 µm.

The particle size of the present emulsions may be measured by laser diffraction. Suitable laser diffraction techniques are well known in the art. The particle size is obtained from a particle size distribution (PSD). The PSD can be determined on a volume, surface, and length basis. The volume particle size is equal to the diameter of the sphere that has the same volume as a given particle. The term Dv represents the average volume particle size of the polynuclear microcapsules. Dv 0.5 is the particle size measured in volume corresponding to 50% of the cumulative particle population. In other words if Dv 0.5 = 10 µm, 50% of the particle have an average volume particle size below 10 µm and 50% of the particle have a volume average particle size above 10 µm. Unless indicated otherwise all average volume particle sizes are calculated using Dv 0.5.

The amount of water added in step II) can vary from 5 to 700 parts per 100 parts by weight of the mixture from step I. The water is added to the mixture from step I at such a rate so as to form an emulsion of the mixture of step I. While this amount of water can vary depending on the selection of the amount of polydialkylsiloxane and aminofunctional organopolysiloxane present and the specific quaternary ammonium surfactant used, generally the amount of water is from 5 to 700 parts per 100 parts by weight of the step I mixture, alternatively from 5 to 100 parts per 100 parts by weight of the step I mixture, or alternatively from 5 to 70 parts per 100 parts by weight of the step I mixture.

The water added to the mixture from step I may be done in incremental portions, whereby each incremental portion comprises less than 30 weight % of the mixture from step I and each incremental portion of water is added successively to the previous after the dispersion of the previous incremental portion of water, wherein sufficient incremental portions of water are added to form an emulsion.

Step III) of the present process is optional and involves further shear mixing of the formed emulsion. The mixing may be accomplished by any of the mixing techniques as described above.

Step IV) in the present process involves adding a sufficient amount of an acid of the formula H⁺ X⁻ to provide the emulsion with a neutral pH, where X is the same counter ion used in the quaternary ammonium surfactant as component B). As used herein "neutral pH" means the emulsion has a pH in the range of 6 to 8, alternatively, 6.5 to 7.5, or alternatively ranging from 6.8 to 7.2. The pH of the emulsion may be measured with a pH meter. The acid of the formula H⁺ X⁻ is typically post added to the emulsion as a dilute aqueous solution prior to other additives. As used herein, "a dilute aqueous solution" may contain 0.1 to 50 weight (wt) percent, alternatively 1 to 40 wt percent, alternatively 5 to 30 wt percent, alternatively 5 to 20 wt percent of the H⁺ X⁻ acid dissolved in water. The dilute aqueous solution is added to the emulsion typically with sufficient stirring to ensure uniform mixing.

In one example, coco trimethylammonium methosulfate is used as quaternary ammonium salt to prepare the emulsion, and a sufficient amount of a 10 wt % aqueous solution of methanesulfonic acid is used in step IV to provide a neutral pH.

Additional additives and components may also be included in the emulsion compositions, such as preservatives, freeze/thaw additives, and various thickeners.

Also disclosed herein are emulsions having an octamethylcyclotetrasiloxane and decamethylcyclopentasiloxane content that is less than 1 weigh percent of the total silicone emulsion. The cyclic siloxane content (that is octamethylcyclotetrasiloxanes (D₄) and decamethylcyclopentasiloxanes (D₅)) may be determined by harvesting the silicone phase of the emulsions with a mixture of polar and nonpolar organic solvents. The solvents containing any cyclic siloxanes can then be analyzed using common gas chromatography techniques.

The present emulsions may be formulated into personal care products. The personal care compositions may be in the form of a cream, a gel, a powder, a paste, or a freely pourable liquid. Generally, such compositions can generally be prepared at room temperature if no solid materials at room temperature are present in the compositions, using simple propeller mixers, Brookfield counter-rotating mixers, or homogenizing mixers. No special equipment or processing conditions are typically required. Depending on the type of form made, the method of preparation will be different, but such methods are well known in the art.

The personal care products may be functional with respect to the portion of the body to which they are applied, cosmetic, therapeutic, or some combination thereof. Conventional examples of such products include antiperspirants and deodorants, skin care creams, skin care lotions, moisturizers, facial treatments such as acne or wrinkle removers, personal and facial cleansers, bath oils, perfumes, colognes, sachets, sunscreens, pre-shave and after-shave lotions, shaving soaps, and shaving lathers, hair shampoos, hair conditioners, hair colorants, hair relaxants, hair sprays, mousses, gels, permanents, depilatories, and cuticle coats, make-ups, color cosmetics, foundations, concealers, blushes, lipsticks, eyeliners, mascara, oil removers, color cosmetic removers, and powders, medicament creams, pastes or sprays including antiacne, dental hygienic, antibiotic, healing promotive, nutritive, which may be preventative and/or therapeutic. In general the personal care products may be formulated with a carrier that permits application in any conventional form, including but not limited to liquids, rinses, lotions, creams, pastes, gels, foams, mousses, ointments, sprays, aerosols, soaps, sticks, soft solids, solid gels, and gels. What constitutes a suitable carrier is readily apparent to one of ordinary skill in the art.

The compositions disclosed herein can be used by the standard methods, such as applying them to the human body, e.g. skin or hair, using applicators, brushes, applying by hand, pouring them and/or possibly rubbing or massaging the composition onto or into the body. Removal methods, for example for color cosmetics are also well known standard methods, including washing, wiping, and peeling. For use on the skin, the compositions disclosed herein may be used in a conventional manner for example for conditioning the skin. An effective amount of the composition for the purpose is applied to the skin. Such effective amounts generally range from 1mg/cm² to 3 mg/cm². Application to the skin typically includes working the composition into the skin. This method for applying to the skin comprises the steps of contacting the skin with the composition in an effective amount and then rubbing the composition into the skin. These steps can be repeated as many times as desired to achieve the desired benefit.

The use of the compositions disclosed herein on hair may use a conventional manner for conditioning hair. An effective amount of the composition for conditioning hair is applied to the hair. Such effective amounts generally range from 0.5 g to 50 g, preferably from 1 g to 20 g. Application to the hair typically includes working the composition through the hair such that most or all of the hair is contacted with the composition. This method for conditioning the hair comprises the steps of applying an effective amount of the hair care composition to the hair, and then working the composition through the hair. These steps can be repeated as many times as desired to achieve the desired conditioning benefit.

### EXAMPLES

The following examples are included to further illustrate the invention. All percentages are in wt. %. All measurements were conducted at 23°C unless indicated otherwise. Rotational viscosities were determined using a *Brookfield Cone and Plate Viscometer Model DVT,* cone #CP 52, operating at 0.5 rpm.

### Example 1

First, 37.81 grams of polydimethylsiloxane (Dow Corning® 200 Fluid) having a viscosity of 600,000 mm²/s at 23°C (cS) was added to a Max 60 dental mixer cup. Then, 4.2 g of Dow Corning® 2-8566 Amino Fluid (a trimethylsiloxy terminated, dimethyl, methyl(aminoethylaminoisobutyl) polysiloxane, having a random distribution of two mole percent of silicon atoms substituted with methyl(aminoethylaminoisobutyl) polysiloxane functionality and of sufficient molecular weight to provide a rotational viscosity (as determined by a Brookfield Cone and Plate Viscometer Model DVT, cone #CP 52, at 0.5 rpm) of 3,000 mPa·s (cP) was added. The silicone fluids were blended using a DAC 250 SpeedMixer TM (FlackTek Inc.). Once homogeneous, 0.6 grams of Lutensol ® XP 79 (BASF), 1.04 grams Luviquat® Mono LS (BASF), and 1.48 grams of water were added. Mixing with the SpeedMixerTM yielded a white opaque emulsion that was highly viscous. Subsequently, the emulsion was diluted with 14.5 grams of deionized water with mixing again from the SpeedMixerTM. Neutralization was accomplished with 0.51 grams of a 10% solution of methanesulfonic acid to yield a pH of about 7. The particle size was measured using a Mastersizer 2000 (Malvern Instruments Ltd.). At the 50^{th} percentile the particle size was 1.906 micrometers while the particle size at the 90^{th} percentile was 3.426 micrometers.

After 7 days at 22C, an aliquot of the emulsion from Example 1 was subjected to a mixture of polar and nonpolar organic solvents to harvest the internal phase of the emulsion. Using gas chromatography the solvents were analyzed for octamethylcyclotetrasiloxane (D4) and decamethylcyclopentasiloxane (D5) with values in the emulsion of 0.06 wt. % and 0.06 wt. %, respectively.

Following aging at 50°C for 92 days and then subjected to a mixture of polar and nonpolar organic solvents to harvest the internal phase of the emulsion, the wt. % of D4 was found to be 0.07 wt. %, while the wt. % of D5 in the emulsion was found to be 0.08 wt. %. The bulk emulsion viscosity and stability were typical of stable, non-neutralized emulsion samples.

### Comparative Example 1

### Luviquat with no Neutralization Acid

First, 63.01 grams of polydimethylsiloxane (Dow Corning® 200 Fluid) having a viscosity of 600,000 mm²/s at 23C (cS) was added to a Max 60 dental mixer cup. Then, 7.05 g. of Dow Corning® 2-8566 Amino Fluid (a trimethylsiloxy terminated, dimethyl, methyl(aminoethylaminoisobutyl) polysiloxane, having a random distribution of two mole percent of silicon atoms substituted with methyl(aminoethylaminoisobutyl) polysiloxane functionality and of sufficient molecular weight to provide a rotational viscosity (as determined by a Brookfield Cone and Plate Viscometer Model DVT, cone #CP 52, at 0.5 rpm) of 3,000 mPa·s (cP) was added. The silicone fluids were blended using a DAC 250 SpeedMixer TM (FlackTek Inc.). Once homogeneous, 1.04 grams of Lutensol ® XP 79 (BASF), 1.70 grams Luviquat® Mono LS (BASF), and 2.55 grams of water were added. Mixing with the SpeedMixerTM yielded a white opaque emulsion that was highly viscous. Subsequently, the emulsion was diluted with 24.50 grams of deionized water with mixing again from the SpeedMixerTM. No neutralization was done to maintain a pH of about 8-8.5. The particle size was measured using a Mastersizer 2000 (Malvern Instruments Ltd.). At the 50^{th} percentile the particle size was 2.196 micrometers while the particle size at the 90^{th} percentile was 3.426 micrometers.

After 7 days at 22°C, an aliquot of the emulsion from Example 1 was subjected to a mixture of polar and nonpolar organic solvents to harvest the internal phase of the emulsion. Using gas chromatography the solvents were analyzed for octamethylcyclotetrasiloxane (D4) and decamethylcyclopentasiloxane (D5) with values in the emulsion of 0.04 wt. % and 0.05 wt. %, respectively.

Following aging at 50°C for 50 days and then subjected to a mixture of polar and nonpolar organic solvents to harvest the internal phase of the emulsion, the wt. % of D4 was found to be 0.22 wt. %, while the wt. % of D5 in the emulsion was found to be 0.09 wt. %. The bulk emulsion viscosity and stability were typical of stable, non-neutralized emulsion samples.

### Comparative Example 2

### Luviquat with Different Counterion Acid Neutralization

First, 63.05 grams of polydimethylsiloxane (Dow Corning® 200 Fluid) having a viscosity of 600,000 mm2/s at 23C (cS) was added to a Max 60 dental mixer cup. Then, 7.00 g. of Dow Corning® 2-8566 Amino Fluid (a trimethylsiloxy terminated, dimethyl, methyl(aminoethylaminoisobutyl) polysiloxane, having a random distribution of two mole percent of silicon atoms substituted with methyl(aminoethylaminoisobutyl) polysiloxane functionality and of sufficient molecular weight to provide a rotational viscosity (as determined by a Brookfield Cone and Plate Viscometer Model DVT, cone #CP 52, at 0.5 rpm) of 3,000 mPa·s (cP) was added. The silicone fluids were blended using a DAC 250 SpeedMixer TM (FlackTek Inc.). Once homogeneous, 0.65 grams of Renex 36 (Croda), 1.72 grams Luviquat® Mono LS (BASF), and 2.56 grams of water were added. Mixing with the SpeedMixerTM yielded a white opaque emulsion that was highly viscous. Subsequently, the emulsion was diluted with 24.38 grams of deionized water with mixing again from the SpeedMixerTM. Neutralization was accomplished with 1.01 grams of a 10% solution of acetic acid to yield a pH of about 7. The particle size was measured using a Mastersizer 2000 (Malvern Instruments Ltd.). At the 50^{th} percentile the particle size was 2.124 micrometers while the particle size at the 90^{th} percentile was 3.627 micrometers.

Following aging at 50°C for 50 days and then subjected to a mixture of polar and nonpolar organic solvents to harvest the internal phase of the emulsion, the wt. % of D4 was found to be 0.06 wt. %, while the wt. % of D5 in the emulsion was found to be 0.05 wt. %.

The bulk emulsion viscosity was significantly thinner than typical stable, non-neutralized or methane sulfonic acid (MSA) stabilized emulsions (7,400 mPa·s (cP) vs. 13,600 mPa·s and 13,400 mPa·s, respectively). Following aging at 50°C for 19 days the emulsion phase separated. The same observation was made for neutralization with other carboxylic acids, such as lactic acid, used to neutralize the emulsion.

## Claims

1. A process for preparing an aminofunctional silicone emulsion comprising:
I) forming a mixture of;
A) 100 parts of a polydialkylsiloxane having a viscosity of
at least 50,000 mm²/s at 23°C as determined by a Brookfield Cone and Plate Viscometer Model DVT, cone #CP 52, at 0.5 rpm, and
B) 0.1 to 100 parts of an aminofunctional organopolysiloxane having an average formula;
[R₃SiO_{1/2}][R₂SiO_{2/2}]ₐ[RR^{N}SiO_{2/2}]_{b}[R₃SiO_{1/2}]
where; a is 1-1000, b is 1-100,
R is independently a monovalent organic group, and
R^{N} is an amino functional group,
II) admixing to the mixture of step I;
C) 0.1 to 50 parts of a halide free quaternary ammonium surfactant of the
formula; R⁵ₐR⁶₍₄₋ₐ)N⁺ X⁻, wherein
a varies from 1 to 4,
R⁵ is an organic group containing at least 10 carbon atoms,
R⁶ is independently a hydrocarbon group containing 1 to 20 carbon atoms, X is a halide free counter ion selected from methosulfate, and
a sufficient amount of water to form an emulsion,
III) optionally, further shear mixing the emulsion,
IV) adding a sufficient amount of an acid of the formula H⁺ X⁻ to provide the emulsion with a neutral pH in the range of 6.5 to 7.5 at 23°C, where X is the same counter ion used in the quaternary ammonium surfactant.

2. The process of claim 1 wherein the polydialkylsiloxane is a trimethylsiloxy terminated polydimethylsiloxane having a viscosity of at least 100,000 mm²/s at 23°C.

3. The process of claim 1 or 2 wherein R is methyl and the amino functional group is -CH₂CH(CH₃)CH₂NHCH₂CH₂NH₂.

4. The process of any one of the above claims where R⁵ is lauryl, cetyl, coco, stearyl, tallow, cocoyl, lauroyl, palmitoyl, myristoyl or stearoyl.

5. The process of claim 4 where R⁶ is methyl or ethyl.

6. The process of any one of the above claims wherein step II) further comprises adding D) a nonionic surfactant.

7. The process of claim 6 wherein the nonionic surfactant is an alkyl polyethylene glycol ether based on C10-Guerbet alcohol.

## Patentansprüche

1. Ein Verfahren zum Herstellen einer aminofunktionellen Silikonemulsion, beinhaltend:
I) Bilden einer Mischung von:
A) 100 Teilen eines Polydialkylsiloxans mit einer Viskosität von mindestens 50 000 mm²/s bei 23 °C, wie durch ein Kegel-Platte-Viskosimeter von Brookfield, Modell DVT, Cone #CP 52, bei 0,5 U/min bestimmt, und
B) 0,1 bis 100 Teilen eines aminofunktionellen Organopolysiloxans mit einer folgenden allgemeinen Formel:
[R₃SiO_{1/2}][R₂SiO_{2/2}]ₐ[RR^{N}SiO_{2/2}]_{b}[R₃SiO_{1/2}]
wobei a 1-1000 ist, b 1-100 ist,
R unabhängig eine monovalente organische Gruppe ist und
R^{N} eine aminofunktionelle Gruppe ist,
II) Beimischen, zu der Mischung aus Schritt I, von:
C) 0,1 bis 50 Teilen eines halogenidfreien quaternären Ammoniumtensids der Formel R⁵ₐR⁶₍₄₋ₐ₎N⁺X⁻, wobei
a von 1 bis 4 variiert,
R⁵ eine organische Gruppe ist, die mindestens 10 Kohlenstoffatome enthält,
R⁶ unabhängig eine Kohlenwasserstoffgruppe ist, die 1 bis 20 Kohlenstoffatome enthält, X ein halogenidfreies Gegenion ist, ausgewählt aus Methosulfat, und
einer ausreichenden Menge von Wasser, um eine Emulsion zu bilden,
III) optional weiteres Schermischen der Emulsion,
IV) Zugeben einer ausreichenden Menge einer Säure der Formel H⁺ X⁻, um der Emulsion bei 23 °C einen neutralen pH-Wert in dem Bereich von 6,5 bis 7,5 zu verleihen, wobei X das gleiche Gegenion wie das in dem quaternären Ammoniumtensid verwendete ist.

2. Verfahren gemäß Anspruch 1, wobei das Polydialkylsiloxan ein trimethylsiloxyterminiertes Polydimethylsiloxan mit einer Viskosität von mindestens 100 000 mm²/s bei 23 °C ist.

3. Verfahren gemäß Anspruch 1 oder 2, wobei R Methyl ist und die aminofunktionelle Gruppe -CH₂CH(CH₃)CH₂NHCH₂CH₂NH₂ ist.

4. Verfahren gemäß einem der vorhergehenden Ansprüche, wobei R⁵ Lauryl, Cetyl, Coco, Stearyl, Talg, Cocoyl, Lauroyl, Palmitoyl, Myristoyl oder Stearoyl ist.

5. Verfahren gemäß Anspruch 4, wobei R⁶ Methyl oder Ethyl ist.

6. Verfahren gemäß einem der vorhergehenden Ansprüche, wobei Schritt II) ferner das Zugeben D) eines nichtionischen Tensids beinhaltet.

7. Verfahren gemäß Anspruch 6, wobei das nichtionische Tensid ein Alkylpolyethylenglycolether auf der Basis von C10-Guerbet-Alkohol ist.

## Revendications

1. Un procédé pour préparer une émulsion de silicone aminofonctionnelle comprenant :
I) la formation d'un mélange de :
A) 100 parties d'un polydialkylsiloxane ayant une viscosité d'au moins 50 000 mm²/s à 23 °C telle que déterminée par un viscosimètre à cône/plateau Brookfield, Modèle DVT, cône #CP 52, à 0,5 tr/min, et
B) 0,1 à 100 parties d'un organopolysiloxane aminofonctionnel ayant une formule moyenne :
[R₃SiO_{1/2}][R₂SiO_{2/2}]ₐ[RR^{N}SiO_{2/2}]_{b}[R₃SiO_{1/2}]
où : a est 1 à 1 000, b est 1 à 100,
R est indépendamment un groupe organique monovalent, et
R^{N} est un groupe amino fonctionnel,
II) l'admixtion au mélange de l'étape I de :
C) 0,1 à 50 parties d'un tensioactif ammonium quaternaire exempt d'halogénure de la formule : R⁵ₐR⁶₍₄₋ₐ₎N⁺ X⁻, dans laquelle
a varie de 1 à 4,
R⁵ est un groupe organique contenant au moins 10 atomes de carbone,
R⁶ est indépendamment un groupe hydrocarboné contenant 1 à 20 atomes de carbone, X est un contre-ion exempt d'halogénure sélectionné dans le méthosulfate, et
une quantité suffisante d'eau pour former une émulsion,
III) facultativement, en sus le mélange par cisaillement de l'émulsion,
IV) l'ajout d'une quantité suffisante d'un acide de la formule H⁺ X- pour fournir à l'émulsion un pH neutre dans la gamme allant de 6,5 à 7,5 à 23 °C, où X est le même contre-ion utilisé dans le tensioactif ammonium quaternaire.

2. Le procédé de la revendication 1 dans lequel le polydialkylsiloxane est un polydiméthylsiloxane à terminaison triméthylsiloxy ayant une viscosité d'au moins 100 000 mm²/s à 23 °C.

3. Le procédé de la revendication 1 ou de la revendication 2 dans lequel R est un méthyle et le groupe amino fonctionnel est -CH₂CH(CH₃)CH₂NHCH₂CH₂NH₂.

4. Le procédé de n'importe laquelle des revendications ci-dessus où R⁵ est un lauryle, un cétyle, un coco, un stéaryle, un suif, un cocoyle, un lauroyle, un palmitoyle, un myristoyle ou un stéaroyle.

5. Le procédé de la revendication 4 où R⁶ est un méthyle ou un éthyle.

6. Le procédé de n'importe laquelle des revendications ci-dessus dans lequel l'étape II) comprend en sus l'ajout de D) un tensioactif non ionique.

7. Le procédé de la revendication 6 dans lequel le tensioactif non ionique est un éther d'alkyle de polyéthylèneglycol à base d'alcool de Guerbet en C10.
